# EUROPÄISCHE PATENTANMELDUNG

(11) **EP 1 319 388 A2**
(43) Veröffentlichungstag der Anmeldung: **18.06.2003**
(21) Anmeldenummer: 02026709.2
(22) Anmeldetag: 30.11.2002
(51) Int. Cl.: A61K 7/11

(54) **Haarfestiger mit nichtionischem Filmpolymer**

(30) Priorität: 12.12.2001 DE 10161000
(71) Anmelder: Beiersdorf AG, 20245 Hamburg (DE)
(72) Erfinder: Primmel, Bettina, 20146 Hamburg (DE); Liebelt, Kerstin, 22529 Hamburg (DE)

(57) **Zusammenfassung**

Haarfestiger enthaltend
a) mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-%
b) mindestens ein nichtionisches Polymer in einer Konzentration von 0,1 bis 10 Gewichts-%,
jeweils bezogen auf das Gesamtgewicht der Zubereitung.

## Beschreibung

Die vorliegende Erfindung betrifft Haarfestiger enthaltend mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-% sowie mindestens einem nichtionischen Polymer in einer Konzentration von 0,1 bis 10 Gewichts-%, jeweils bezogen auf das Gesamtgewicht der Zubereitung und deren Verwendung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich unterscheiden sich aber in ihrer Aufgabe und Anwendung.

Schaumfestiger (auch Haarschaumfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die beiden Grundbausteine von Haarfestigern sind die Filmbildner und das Lösungsmittel. Als Filmbildner werden meist Mischpolymerisate aus Vinylpyrrolidon und Vinylacetat oder neutralisierter Crotonsäure eingesetzt. Filmbildner bleiben nach dem Verdunsten des Lösungsmittels (meist Wasser und/oder Ethanol) als Film auf dem Haar haften und fixieren dessen Form. Haarschaumfestiger enthalten zusätzlich Treibmittel wie Propan, n-Butan und/oder Isobutan.

Je nach gewünschter Festigkeit der Frisur werden unterschiedlich stark festigende Filmbildner in unterschiedlichen Mengenanteilen eingesetzt. Man unterscheidet daher Haarfestiger mit schwacher, normaler und starker Festigung sowie Festiger für normales, trockenes und fettiges Haar [W. Umbach (Hrsg.): Kosmetik, Entwicklung, Herstellung und Anwendung kosmetischer Mittel, 2. Aufl., Thieme Verlag, Stuttgart, 1995].

Herkömmliche Haarfestiger aus einer Kombination von nichtionischen und kationischen Polymeren haben den Nachteil, dass sie den Anforderungen bezüglich ihrer Festigungseigenschaften für die Frisur (auch Langzeithalt genannt), nur unzureichend gerecht werden, wenn gleichzeitig die Kämmbarkeit und der "Griff" des nassen und trockenen Haares den Verbraucherwünschen entsprechen soll. Insbesondere das Entstehen von sogenannten Filmplaken, das heißt Rückständen, die beim Kämmen auf Schulter und Kleidung rieseln und den Anwender ungepflegt erscheinen lassen, läßt sich mit Produkten des Standes der Technik nur unzureichend unterdrücken, wenn eine hohe Festigung erzielt werden soll.

Herkömmliche Haarfestiger basierend auf nichtionischen und kationischen Polymeren erreichen nur begrenzte Festigunggrade.
Werden die Konzentrationen der Polymere in den Zubereitungen zur Erzielung sehr starker Festigungsgrade wesentlich erhöht, resultieren daraus als negative Begleiterscheinungen eine starke Rückstandsbildung sowie eine spröde Filmtextur auf dem Haar, die aus Konsumentensicht nicht akzeptabel erscheint.

Zur Erzielung akzeptabler Filmelastizitäten und Griffeigenschaften können zwar Stylingformulierungen auf Basis eines Gemisches von nichtionischen und kationischen Polymeren mit Weichmachern kombiniert werden, diese reduzieren aber gleichzeitig die ohnehin begrenzten Festigungseigenschaften der Formulierung.

Es war daher die Aufgabe der vorliegenden Erfindung, den Nachteilen des Standes der Technik abzuhelfen und Haarfestigungszubereitungen zu entwickeln, die dem Haar eine hohe Festigkeit und hohe Filmelastizität geben. Der Haarfestiger sollte pflegend wirken und dabei das Auftreten von Filmplaken unterdrücken.

Überraschend wird die Aufgabe gelöst durch Haarfestiger enthaltend
a) mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-%
b) mindestens ein nichtionisches Polymer in einer Konzentration von 0,1 bis 10 Gewichts-%,
   jeweils bezogen auf das Gesamtgewicht der Zubereitung.

Durch erfindungsgemäße Haarfestiger bekommt das Haar eine sehr starke Festigung und insbesondere eine verbesserte Filmelastizität, bei gleichzeitiger Pflege des Haares. Das Produkterlebnis wird für den Verbraucher deutlich verbessert. Das Produkt hinterläßt darüber hinaus keine Rückstände im Haar.

Zwar sind Terpolymere aus Vinylpyrrolidon, Dimethylaminopropylmethacrylamid und quaternisiertem Alkyldimethylaminopropylmethacrylamid und ihre Verwendung in haarfestigenden Mitteln, insbesondere in Aerosol- und Non-Aerosol-Festigungsprodukten in der WO 00 68 282 beschrieben. Die in dieser Schrift offenbarten Zubereitungen zeichnen sich zwar durch gute Filmeigenschaften wie geringe Klebrigkeit und hohe Feuchtigkeitsresistenz auf, ihre Festigungseigenschaften, lassen jedoch zu wünschen übrig.

Erfindungsgemäß besonders vorteilhaft ist die Konzentration eines Terpolymers aus Vinylpyrrolidon, Dimethylaminopropylmethacrylamid und quaternisiertem Alkyldimethylaminopropylmethacrylamid von 0,1 bis 10 Gewichts-% und insbesondere von 0,25 bis 2,5 Gewichts-%, jeweils bezogen auf das Gesamtgewicht der Zubereitung.

Die erfindungsgemäß vorteilhaften Terpolymere bestehen aus Vinylpyrrolidon, Dimethylaminopropylmethacrylamid und Alkyldimethylaminopropylmethacrylamidoammoniumsalzen.

Alle in der WO 00 68 282 offenbarten Terpolymere sind erfindungsgemäß vorteilhaft.

Ganz besonders vorteilhaft ist dabei das Terpolymer aus Vinylpyrrolidon, Dimethylaminopropylmethacrylamid und Lauryldimethylaminopropylmethacrylamidoammoniumchlorid (z.B. Styleze W-20 der Firma ISP).

Erfindungsgemäß besonders vorteilhaft ist die Konzentration, mindestens eines nichtionischen Polymers von 0,1 bis 10 Gewichts-% und insbesondere 1 bis 5 Gewichts-%, jeweils bezogen auf das Gesamtgewicht der Zubereitung.

Es ist erfindungsgemäß vorteilhaft, das nichtionische Polymer aus der Gruppe der folgenden Verbindungen zu wählen:
- Homopolymere des N-Vinylpyrrolidons [z.B. LUVISKOL K (BASF) und PVP-K (ISP), mit verschiedenen Molmassen, als Pulver, wässrige oder wässrig/alkoholische Lösung]. Besonders bevorzugt ist das LUVISKOL K30.
- Homopolymere des N-Vinylcaprolactams [z.B. LUVISKOL Plus (BASF)].
- Homopolymere des N-Vinylformamids.
- Copolymere aus N-Vinylpyrrolidon und Vinylacetat und/oder Vinylpropionat [LUVISKOL VA (BASF) und PVP/VA (ISP)]. Besonders bevorzugt sind dabei LUVISKOL VA37E und/oder LUVISKOL VA64W.
- Terpolymere aus N-Vinylpyrrolidon, Vinylacetat und Vinylpropionat [z.B. LUVISKOL VAP 343 (BASF)].
- Terpolymere aus N-Vinylpyrrolidon, Acrylamid und Vinylalkohol.

Erfindungsgemäß besonders vorteilhaft sind dabei als nichtionische Polymere Copolymere aus N-Vinylpyrrolidon und Vinylacetat, insbesondere mit einem Verhältnis von Vinylpyrrolidon zu Vinylacetat von 6:4 (z.B. LUVISKOL VA64W).

Eine besonders bevorzugte Ausführungsform der erfindungsgemäßen Haarfestiger ist ihr Vorliegen in Form einer wässrig und/oder alkoholischen Lösung.

Die erfindungsgemäßen Haarfestiger können vorteilhaft mit einem Treibgas aufgeschäumt werden. Dabei ist es vorteilhaft, das Treibgas in einer Menge von 0,5 bis 20 Gewichts-% und besonders bevorzugt in einer Konzenrtation von 8 bis 11 Gewichts-%, jeweils bezogen auf das Gesamtgewicht der Formulierung einzusetzen.

Die erfindungsgemäß bevorzugten Treibgase sind Propan, Isobutan und n-Butan sowie deren Mischungen. Aber auch Druckluft, Kohlendioxid, Stickstoff, Stickstoffdioxid und Dimethylether sowie Mischungen all dieser Gase sind erfindungsgemäß vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Antioxidanien, Bakterizide, Parfüme, Emulgatoren, weichmachende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Die erfindungsgemäßen Haarfestiger können vorteilhafter Weise in einer Pumpsprayoder Aerosolverpackung vorliegen, welche als Schaumspender dienen und aus denen heraus die Haarfestiger angewendet werden können.

Dem entsprechend sind Schaumspender auf Pumpspay- oder Aerosolverpackungsbasis, welche erfindungsgemäße Haarfestiger enthalten ebenso Bestandteil der Erfindung.

Aber auch die Aufbewahrung in Quetschflaschen und anderen in der Haarpflege eingesetzten Verpackungsbehältnissen in denen die erfindungsgemäßen Haarfestiger in Form von Lösungen oder Emulsionen vorliegen und aus diesen heraus angewendet werden können, ist erfindungsgemäß vorteilhaft.

Außerdem ist erfindungsgemäß die Verwendung der erfindungsgemäßen Haarfestiger als Schaumfestiger, Flüssigfestiger, Stylinggel oder Schaumaerosol. Die erfindungsgemäßen Haarfestiger können jedoch auch als Emulsion oder Lösung vorliegen.

Die Verwendung nichtionischer Polymerer zur Verbesserung der Festigkeit, der Filmelastizität und der Griffeigenschaften des Haars sowie zur Reduzierung der Entstehung von Filmplaken nach der Anwendung von kosmetischen und/oder dermatologischen Zubereitungen für das Haar enthaltend Terpolymer aus Vinylpyrrolidon, Dimethylaminopropylmethacrylamid und quarternisiertem Dimethylaminopropylmethacrylamid ist ebenso erfindungsgemäß wie die Verwendung von Terpolymer aus Vinylpyrrolidon, Dimethylaminopropylmethacrylamid und quaternisiertem Alkyldimethylaminopropylmethacrylamid zur Verbesserung der Festigkeit, der Filmelastizität und der Griffeigenschaften des Haars sowie zur Reduzierung der Entstehung von Filmplaken nach der Anwendung von kosmetischen und/oder dermatologischen Zubereitungen für das Haar enthaltend nichtionische Polymere.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Schaumfestiger der Festigungsstärke ultra starker Halt** | | | | |
|---|---|---|---|---|
| | **1** | **2** | **3** | **4** |
| Vinylpyrrolidon, Dimethylaminopropylmethacrylamid, Lauryldimethylaminopropylmethacrylamidoammoniumchlorid, Styleze W-20 der Firma ISP | 0,5 | 1 | 1,5 | |
| Vinylpyrrolidon /Vinylimidazolinium-methochlorid Copolymer, Luviquat FC 370, BASF | | | | 2 |
| Vinylpyrrolidon /Vinylacetat Copolymer, LUVISKOL VA64W, BASF | 4,5 | 3,5 | 3,0 | 4,5 |
| Cetyltrimethylammoniumchlorid | 0,5 | 0,4 | 0,3 | 0,3 |
| Parfüm, Lösungsvermittler, pH-Einstellung, Konservierung | | | | |
| Propan/Butan | 10 | 10 | 10 | 10 |
| Wasser | ad 100 | ad 100 | ad 100 | ad 100 |

| **Styling-Gele der Festigungsstärke ultra starker Halt** | | | | |
|---|---|---|---|---|
| | **1** | **2** | **3** | **4** |
| Vinylpyrrolidon, Dimethylaminopropylmethacrylamid, Lauryldimethylaminopropylmethacrylamidoammoniumchlorid, Styleze W-20 der Firma ISP | 0,5 | 1 | 1,5 | |
| Vinylpyrrolidon /dimethylaminoethyl-Methacrylat Copolymer quaternisiert, Gafquat 755N, ISP | | | | 2 |
| Vinylpyrrolidon /Vinylacetat Copolymer, LUVISKOL VA64W, BASF | 4,0 | 3,5 | 3,0 | 4,5 |
| Hydroxyethylcellulose | 0,35 | 0,35 | 0,35 | 0,35 |
| Propylenglykol | 0,5 | 0,5 | 0,5 | 0,5 |
| Cetyltrimethylammoniumchlorid | 0,2 | 0,15 | 0,1 | 0,1 |
| Parfüm, Lösungsvermittler, pH-Einstellung, Konservierung | q.s. | q.s. | q.s. | q.s. |
| Propan/Butan | 10 | 10 | 10 | 10 |
| Wasser | ad 100 | ad 100 | ad 100 | ad 100 |

## Patentansprüche

1. Haarfestiger enthaltend
a) mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-%
b) mindestens ein nichtionisches Polymer in einer Konzentration von 0,1 bis 10 Gewichts-%,
jeweils bezogen auf das Gesamtgewicht der Zubereitung.

2. Haarfestiger nach Anspruch 1, **dadurch gekennzeichnet, dass** das nichtionische Polymer gewählt wird aus der Gruppe Homopolymere des N-Vinylpyrrolidons, Homopolymere des N-Vinylcaprolactams, Homopolymere des N-Vinylformamids, Copolymere aus N-Vinylpyrrolidon und Vinylacetat und/oder Vinylpropionat sowie Terpolymere aus N-Vinylpyrrolidon, Vinylacetat, Vinylpropionat, Terpolymere aus N-Vinylpyrrolidon, Acrylamid und Vinylalkohol.

3. Haarfestiger nach einem der Ansprüche 1 oder 2, **dadurch gekennzeichnet, dass** die Zubereitung mit einem Treibgas aufgeschäumt wird.

4. Haarfestiger nach einem der Ansprüche 1 bis 3, **dadurch gekennzeichnet, dass** das Treibgas in einer Menge von 0,5 bis 20 Gewichts-% bezogen auf das Gesamtgewicht der Formulierung eingesetzt wird.

5. Haarfestiger nach einem der Ansprüche 1 bis 4, **dadurch gekennzeichnet, dass** als Treibgas Propan, Isobutan und/oder n-Butan eingesetzt wird.

6. Haarfestiger nach einem der Ansprüche 1 bis 5, **dadurch gekennzeichnet, dass** die Zubereitung weitere Wirk-, Hilfs und/oder Zusatzstoffe enthält.

7. Haarfestiger nach einem der Ansprüche 1 bis 6, **dadurch gekennzeichnet, dass** die Zubereitung in einer Pumpspray- oder Aerosolverpackung als Schaumspender vorliegt, aus der heraus sie angewendet wird.

8. Schaumspender auf Pumpspay- oder Aerosolverpackungsbasis, enthaltend einen Haarfestiger nach einem der Ansprüche 1 bis 6.

9. Verwendung eines Haarfestigers nach einem der vorhergehenden Ansprüche als Schaumfestiger, Flüssigfestiger, Stylinggel oder Schaumaerosol.

10. Verwendung von nichtionischen Polymeren nach Anspruch 1 bis 9 zur Verbesserung der Festigkeit, der Filmelastizität und der Griffeigenschaften des Haars sowie zur Reduzierung der Entstehung von Filmplaken nach der Anwendung von kosmetischen und/oder dermatologischen Zubereitungen für das Haar enthaltend Terpolymer aus Vinylpyrrolidon, Dimethylaminopropylmethacrylamid und quaternisiertem Alkyldimethylaminopropylmethacrylamid.

11. Verwendung von Terpolymer aus Vinylpyrrolidon, Dimethylaminopropylmethacrylamid und quaternisiertem Alkyldimethylaminopropylmethacrylamid zur Verbesserung der Festigkeit, der Filmelastizität und der Griffeigenschaften des Haars sowie zur Reduzierung der Entstehung von Filmplaken nach der Anwendung von kosmetischen und/oder dermatologischen Zubereitungen für das Haar enthaltend nichtionische Polymere.
